# EUROPEAN PATENT APPLICATION

(11) **EP 1 658 950 A1**
(43) Date of publication of application: **24.05.2006**
(21) Application number: 04745255.2
(22) Date of filing: 18.05.2004
(51) Int. Cl.: B29C 47/26

(54) **EXTRUSION MOLDING APPARATUS FOR RESIN MULTI-LAYER TUBE**

(30) Priority: 27.08.2003 JP 2003303096
(71) Applicant: Pla Giken Co., Ltd., Suita-shi, Osaka 564-0051 (JP)
(72) Inventor: KIKUSAWA, Yoshiharu, c/o PLA Giken Co., Ltd., Suita-shi, Osaka 564-0051 (JP)
(74) Representative: Hering, Hartmut
(86) International application number: PCT/JP2004/006656
(87) International publication number: WO 2005/023514

(57) **Abstract**

The respective wall thicknesses of inner and outer layers in a multi-layer tube molded by an extrusion molding apparatus are made more accurate. The extrusion molding apparatus (1) comprises a plurality of extruders (6, 7) for thermally melting and extruding resins (3, 4) of different kinds, and a die (11) formed with an inner layer tube molding passage (9) for passing therethrough the resin (3) extruded from one extruder (6) of these extruders (6, 7) to enable the molding of an inner layer tube (2a), and an outer layer tube molding passage (10) for passing therethrough the resin (4) extruded from the other extruder (7) to enable the molding of an outer layer tube (2b) which is to be externally fitted integrally on the inner layer tube (2a), the die (11) enabling the molding of a multi-layer tube (2) by these inner and outer layer tubes (2a, 2b). Inner and outer extrusion ports (17, 18) constituting the respective front ends of the inner and outer layer tube molding passages (9, 10) are disposed radially close to each other and are opened forwardly from the front end surface (19) of the die (11) and separately from each other.

## Description

### TECHNICAL FIELD

The present invention relates to an extrusion molding apparatus for a resin multi-layer tube, designed to mold a multi-layer tube used as a material for catheters or the like, by causing thermally melted resin being extruded from an extruder to pass through a die.

### BACKGROUND ART

As for said extrusion molding apparatus for a resin multi-layer tube, there has heretofore been one shown in the following Patent Document 1. According to this gazette, said extrusion molding apparatus comprises a plurality of extruders for thermally melting and extruding resins of different kinds, and a die for passing therethrough the resins extruded from these extruders to enable the molding of a multi-layer tube.

More specifically, said die is formed with an inner layer tube molding passage for forwardly passing therethrough the resin extruded from one of said extruders to enable the molding of an inner layer tube, an outer layer tube molding passage for forwardly passing therethrough the resin extruded from the other extruder to enable the molding of an outer layer tube which is to be externally fitted integrally on said inner layer tube, and a merging passage which merges said inner and outer layer tube molding passages together and which has an extrusion port whose front end opens forwardly of said die.

And, during the operation of said extrusion molding apparatus, each extruder is driven to extrude the thermally melted resin therefrom. Thereupon, these resins forwardly pass through said inner and outer layer tube molding passages, whereby said inner and outer tubes are molded. Further, subsequently, these inner and outer layer tubes pass through said merging passage, whereby they are integrally fixed to mold said multi-layer tube.

Patent Document 1: Japanese Patent Laid-Open Gazette No. 2001-88199

### DISCLOSURE OF THE INVENTION

### PROBLEMS TO BE SOLVED BY THE INVENTION

In this connection, in the molding of said multi-layer tube, when said inner and outer layer tubes pass through said merging passage, they press each other. For this reason, it is not easy to ensure that their respective wall thicknesses obtain desired values. Furthermore, the resins which are materials for them differ in hardness at ordinary temperature from each other. For this reason, the harder resin presses the softer resin, tending to unintentionally cause large deformation. Consequently, with the extrusion molding apparatus described above, it is not easy to ensure that the respective wall thicknesses of said inner and outer layer tubes in said multi-layer tube are accurate.

### MEANS FOR SOLVING PROBLEMS

The invention, which has been accomplished with the above in mind, has an object to ensure that the respective wall thicknesses of the inner and outer layers in the multi-layer tube molded by an extrusion molding apparatus are further accurate.

The invention provides an extrusion molding apparatus for a resin multi-layer tube, comprising a plurality of extruders for thermally melting and extruding resins of different kinds, and a die formed with an inner layer tube molding passage for forwardly passing therethrough the resin extruded from one of these extruders to enable the molding of an inner layer tube, and an outer layer tube molding passage for forwardly passing therethrough the resin extruded from the other extruder to enable the molding of an outer layer tube which is to be externally fitted integrally on said inner layer tube, said die enabling the molding of a multi-layer tube by these inner and outer layer tubes, said extrusion molding apparatus for resin multi-layer tube being
characterized in that
inner and outer extrusion ports constituting the respective front ends of said inner and outer layer tube molding passages are disposed radially close to each other and are opened forwardly from the front end surface of the die separately from each other.

In addition, added to said invention, there is provided an extrusion molding apparatus for a resin multi-layer tube, with said die formed with a through-hole longitudinally extending through said die and passing inwardly of said inner layer tube molding passage, said tube being externally fitted on a core material forwardly passing through said through-hole, wherein
said inner extrusion port of said inner layer tube molding passage may be disposed radically close to the front end opening constituting the front end of said through-hole.

Further, in said invention, another die may be installed which is disposed forwardly of said die and having a die hole communicating with said inner and outer extrusion ports.

Further, in said invention, the front vicinity of said outer extrusion port of said outer layer tube molding passage may be radially outwardly opened.

### EFFECTS OF THE INVENTION

The effects of the invention are as follows.

The invention provides an extrusion molding apparatus for a resin multi-layer tube comprising a plurality of extruders for thermally melting and extruding resins of different kinds, and a die provided with an inner layer tube molding passage for forwardly passing therethrough the resin extruded from one of these extruders to enable the molding of an inner layer tube, and an outer layer tube molding passage for forwardly passing therethrough the resin extruded from the other extruder to enable the molding of an outer layer tube which is to be externally fitted integrally on said inner layer tube, said die enabling the molding of a multi-layer tube by these inner and outer layer tubes, wherein
inner and outer extrusion ports constituting the respective front ends of said inner and outer layer tube molding passages are disposed radially close to each other and opened forwardly of the front end surface of the die separately from each other.

For this reason, when each resin is extruded from each extruder by the driving of each said extruder, each resin is passed through each tube molding passage in said die, whereby inner and outer layer tubes are molded. Further, when each said resin is extruded from the inner and outer extrusion ports forwardly of the die, the outer layer tube is externally fitted integrally on said inner layer tube, whereby a multi-layer tube is molded.

Here, as described above, the inner and outer extrusion ports are disposed radially close to each other. For this reason, when each said resin is passed through each tube molding passage and extruded forwardly from the inner and outer extrusion ports, said inner and outer layer tubes immediately after they are extruded forwardly from said inner and outer extrusion ports fit together and smoothly integrated without requiring relatively large radial deformation.

Furthermore, the inner and outer extrusion ports are opened forwardly from the front end surface of said die separately from each other, as described above. For this reason, when said inner and outer layer tubes fit together, these inner and outer layer tubes are suppressed from pressing each other. Consequently, these inner and outer layer tubes are prevented from being unintentionally deformed by their pressing each other.

As a result, the respective wall thicknesses of the inner and outer layer tubes in the multi-layer tube molded by said extrusion molding apparatus can be made more accurate.

In addition, added to the above invention, there is provided an extrusion molding apparatus for a resin multi-layer tube, with said die formed with a through-hole longitudinally extending through said die and passing inwardly of said inner layer tube molding passage, said tube being externally fitted on a core material forwardly passing through said through-hole, wherein
said inner extrusion port of said inner layer tube molding passage may be disposed close to the front end opening radially constituting the front end of said through-hole.

With the arrangement thus made, by the driving of each said extruder, a multi-layer tube is molded as it is extruded from said die, and this multi-layer tube is externally fitted on said core material, so that an intermediate product is molded using this multi-layer tube and the core material.

Here, the inner extrusion port is disposed radially close to said front end opening, as described above. Furthermore, the inner and outer extrusion ports are disposed radially close to each other. For this reason, when said multi-layer tube is extruded forwardly of said die, the inner and outer layer tubes of said multi-layer tube immediately after their extrusion are, without requiring large radial deformation, externally fitted on the core material immediately after slipping out of the front end opening in said through-hole as it forwardly passes through said through-hole.

Consequently, the multi-layer tube in said intermediate product molded by said extrusion molding apparatus can have the respective wall thicknesses of the inner and outer layer tubes made more accurate.

Further, in said invention, another die may be installed which is disposed forwardly of said die and has a die hole for communicating with said inner and outer extrusion ports.

With the arrangement thus made, the multi-layer tube extruded from said die is passed through said die hole, whereby the perfect circularity and radial dimensions can be finally determined. Consequently, the respective wall thicknesses of the inner and outer layer tubes in said multi-layer tube can be made more uniform with respect to each other in any peripheral portion, so that the multi-layer tube can be made more accurate.

Further, in said invention, the front vicinity of said outer extrusion port of said outer layer tube molding passage may be opened radially outward.

With the arrangement thus made, the outer diameter of the multi-layer tube extruded from said die can be optionally set in any section along the longitudinal direction, thus enabling the molding of tubes of various shapes.

### BRIEF DESCRIPTION OF THE DRAWINGS

[Fig. 1] a side sectional view of an extrusion molding apparatus.
[Fig. 2] a partial enlarged sectional view of Fig. 1.
[Fig. 3] a sectional view taken along the line 3-3 in Fig. 1.
[Fig. 4] a sectional view of an intermediate product.
[Fig. 5] a sectional view of another intermediate product.
[Fig. 6] a view corresponding to part of Fig. 2, showing another embodiment.

### DESCRIPTION OF THE REFERENCE CHARACTERS

- 1: Extrusion molding apparatus
- 2: Multi-layer tube
- 2a: Inner layer tube
- 2b: Outer layer tube
- 3: Resin
- 4: Resin
- 6: Extruder
- 7: Extruder
- 9: Tube molding passage
- 10: Tube molding passage
- 11: Die
- 16: Axis
- 17: Extrusion port
- 18: Extrusion port
- 19: Front end surface
- 21: Inflow passage
- 22: Inflow passage
- 24: Through-hole
- 25: Core material
- 26: Front end opening
- 29: Die hole
- 30: Another die
- 34: Flow adjusting valve
- 35: Flow adjusting valve
- 36: Valve main body
- 37: Valve body fit hole
- 38: Axis
- 39: Valve body
- 41: First valve hole
- 42: Second valve hole
- 43: Communication passage
- 44: Opening-degree adjusting valve
- 47: Intermediate molded article

### BEST MODE FOR CARRYING OUT THE INVENTION

A best mode for carrying out the invention to realize an object which, relating to an extrusion molding apparatus for a resin multi-layer tube according to the invention, is to make more accurate the respective wall thicknesses of the inner and outer layers in a multi-layer tube molded by the extrusion molding apparatus, is as follows.

That is, the extrusion molding apparatus comprises a plurality of extruders for thermally melting and extruding resins of different kinds, and a die for passing therethrough the resins extruded from these extruders to enable the molding of a multi-layer tube. Said die is formed with an inner layer tube molding passage for forwardly passing therethrough the resin extruded from one of said extruders to enable the molding of an inner layer tube, and an outer layer tube molding passage for forwardly passing therethrough the resin extruded from the other extruder to enable the molding of an outer layer tube which is to be externally fitted integrally on said inner layer tube, it being possible to mold said multi-layer tube by said inner and outer layer tubes.

Inner and outer extrusion ports constituting the respective front ends of said inner and outer layer tube molding passages are disposed radially close to each other and are opened forwardly of the front end surface of the die separately from each other.

### EMBODIMENTS

To describe the invention in more detail, embodiments thereof will be described with reference to the accompanying drawings.

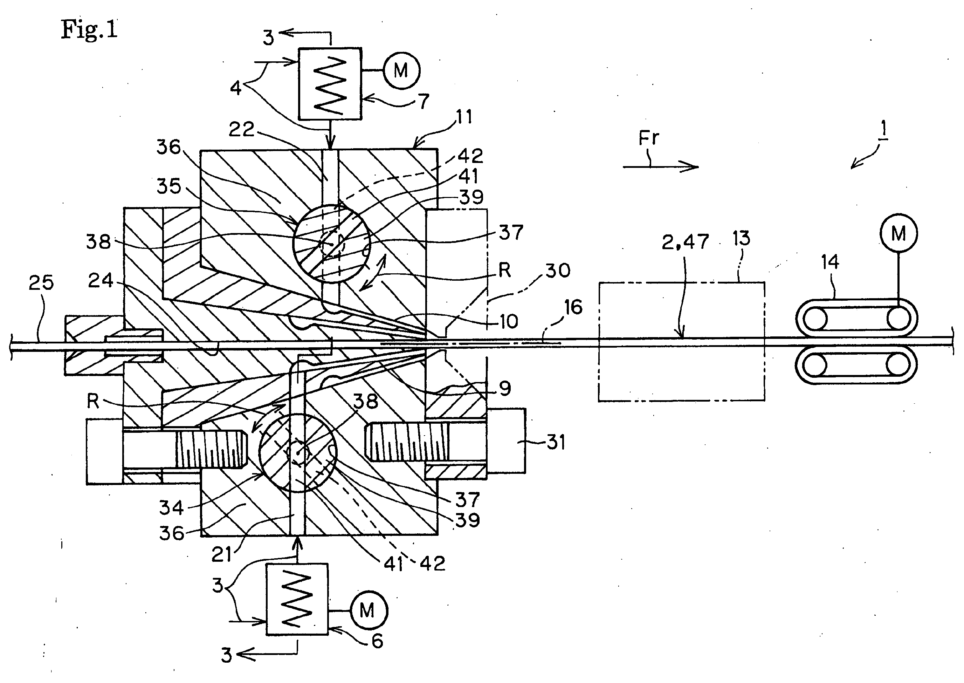

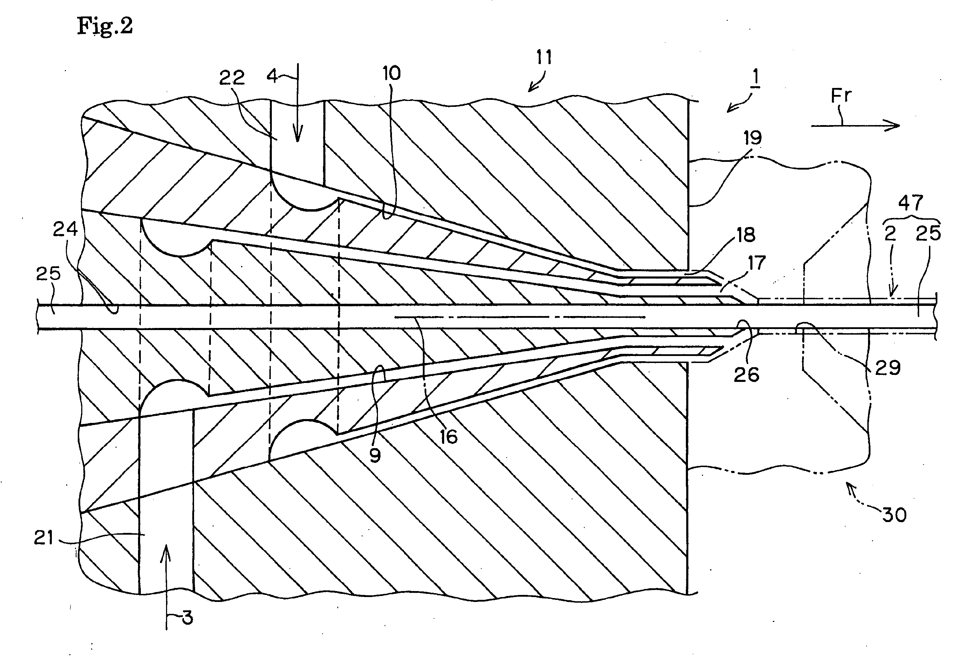

In Figs. 1 - 3, the character 1 denotes an extrusion molding apparatus. This extrusion molding apparatus 1 is used for extrusion-molding a multi-layer tube 2 of circular section made of resin. This multi-layer tube 2 comprises an inner layer tube 2a constituting the inner layer thereof, and an outer layer tube 2b constituting the outer layer of said multi-layer tube 2 and externally fitted on said inner layer tube 2a to be integrally fixed to the outer peripheral surface of this inner layer tube 2a. Said multi-layer tube 2 is used, e.g., as a material for catheters and its outer diameter is 1.0 - 1.5 mm. Further, the arrow Fr in the figure indicates the forward direction of extrusion of the multi-layer tube 2 by said extrusion molding apparatus 1.

The extrusion molding apparatus 1 comprises a plurality (two) of first and second extruders 6 and 7 for thermally melting thermoplastic first and second resins 3 and 4 to enable their extrusion, a die 11 formed with inner and outer layer tube molding passages 9 and 10 through which the first and second resins 3 and 4 extruded from these first and second extruders 6 and 7 are separately forwardly passed to enable the molding of the inner and outer layer tubes 2a and 2b of said multi-layer tube 2, a cold-curing device 13 for cold-curing by water said multi-layer tube 2 molded by being passed through said inner and outer layer tube molding passages 9 and 10, and an electrically driven take-up device 14 for taking up said multi-layer tube 2 cured by this cold-curing device 13, at a predetermined speed (for example, 2.5 - 10 m/min).

Said first and second resins 3 and 4 differ from each other in hardness at ordinary temperature. Further, the thermally melting of said first and second resins 3 and 4 is achieved by heating using a heater. Further, said first and second extruders 6 and 7 are used for rotationally driving screws by an electric motor.

Said die 11 will now be described in more detail. Said inner and outer layer tube molding passages 9 and 10 are each in the form of a forwardly tapering frustum and are disposed on the same axis 16. Further, in the radial direction (orthogonal direction, hereinafter the same) of this axis 16, the inner layer tube molding passage 9 is disposed inwardly of the outer layer tube molding passage 10. The respective front ends of said inner and outer layer tube molding passages 9 and 10 are constituted by inner and outer extrusion ports 17 and 18, these extrusion ports 17 and 18 extending substantially in parallel with said axis 16. These inner and outer extrusion ports 17 and 18 enable said first and second resins 3 and 4 to be extruded forwardly which is outside said die 11. Said inner and outer extrusion ports 17 and 18 are disposed radially of said axis 16 and close to each other. Further, said inner and outer extrusion ports 17 and 18 are partly or wholly opened forwardly from the front end surface 19 of said die 11 and separately from each other.

Said die 11 is formed with first and second inflow passages 21 and 22. These inflow passages 21 and 22 enables the first and second resins 3 and 4 extruded from said first and second extruders 6 and 7 to flow into the respective rears of said inner and outer layer tube molding passages 9 and 10 separately from each other. In this case, the cross-sectional areas of the two inflow passages 21 and 22 are substantially the same. In addition, the cross-sectional area of the first inflow passage 21 may be made larger than that of the second inflow passage, and vise versa.

The first resin 3 extruded from the first extruder 6, which is one extruder 6 of said first and second extruders 6 and 7, is caused to flow into the rear of said inner layer tube molding passage 9 via said first inflow passage 21. And, thereafter, said resin 3 is passed through said inner layer tube molding passage 9 and extruded forwardly of said die 11, whereby said inner layer tube 2a is molded. Further, the second resin 4 extruded from the second extruder 7 which is the other extruder 7 is caused to flow into the rear of said outer layer tube molding passage 10 via said second inflow passage 22. And, thereafter, said resin 4 is passed through said outer layer tube molding passage 10 and extruded forwardly of said die 11, whereby said outer layer tube 2b is molded. In this case, this outer layer tube 2b is externally fitted integrally on said inner layer tube 2a. In other words, said first and second resins 3 and 4 are passed through the inner and outer layer tube molding passages 9 and 10 via said first and second inflow passages 21 and 22, whereby said multi-layer tube 2 is molded.

A through-hole 24 of circular cross-section extending on said axis 16 is formed in said die 11. Said through-hole 24 longitudinally extends through said die 11 and is formed inwardly of said inner layer tube molding passage 9. A core material 25 of circular cross-section made of copper metal is allowed to forwardly pass through said through-hole 24. The inner diameter of said through-hole 24 is substantially equal to the outer diameter of said core material 25. And, the inner layer tube 2a of said multi-layer tube 2 is externally fitted on the core material 25 passing forwardly in said through-hole 24, and said inner layer tube 2a can closely adhere to said core material 25. Further, disposed radially of said axis 16 and in the vicinity of the front end opening 26 constituting the front end of said through-hole 24 is the inner extrusion port 17 of said inner layer tube molding passage 9.

Installed on said axis 16 is another die 30 having a die hole 29 communicating with the extrusion ports 17 and 18 of said tube molding passages 9 and 10. This another die 30 is removably fixed to the front end surface 19 of said die 11 by a fastener 31.

First and second flow adjusting valves 34 and 35 are installed. These first and second flow adjusting valves 34 and 35 make it possible to separately adjust the respective flows per unit time (m³/min: hereinafter referred to simply as flows) of the first and second resins 3 and 4 extruded from said first and second extruders 6 and 7 and passed to said inner and outer layer tube molding passages 9 and 10.

Further, said flow adjusting valves 34 and 35 adjust the degrees of opening of said inflow passages 21 and 22 to make it possible to adjust the flows of the resins 3 and 4. Concretely, said flow adjusting valves 34 and 35 each comprise a valve main body 36 constituted by part of said die 11, a columnar valve body 39 fitted in a circular valve body fit hole 37 formed in said valve main body 36 to divide the longitudinal intermediate portion of each of the inflow passages 21 and 22, in such a manner as to allow its turn R around the axis 38, and an actuator 40, such as an air cylinder, which enables this valve body 39 to make turn R to a predetermined turn position. Said valve bodies 39 are formed with first and second valve holes 41 and 42 radially extending therethrough and independent of each other. Formed in each valve body 39 is a communication passage 43 communicating the intermediate portion of said second valve hole 42 to outside the die 11. Further, there is installed a needle valve type opening degree adjusting valve 44 which makes it possible to manually adjust the degree of opening of said communication passage 43.

The electric motors for said extruders 6 and 7 and take-up device 14, and actuators 40 are connected to an electronic controller and automatically controlled according to a predetermined program. Here, said extruders 6 and 7 are driven such that when the pressures of the resins 3 and 4 immediately after their extrusion have predetermined values, the flows of the resins 3 and 4 extruded from the extruders 6 and 7 are substantially constant.

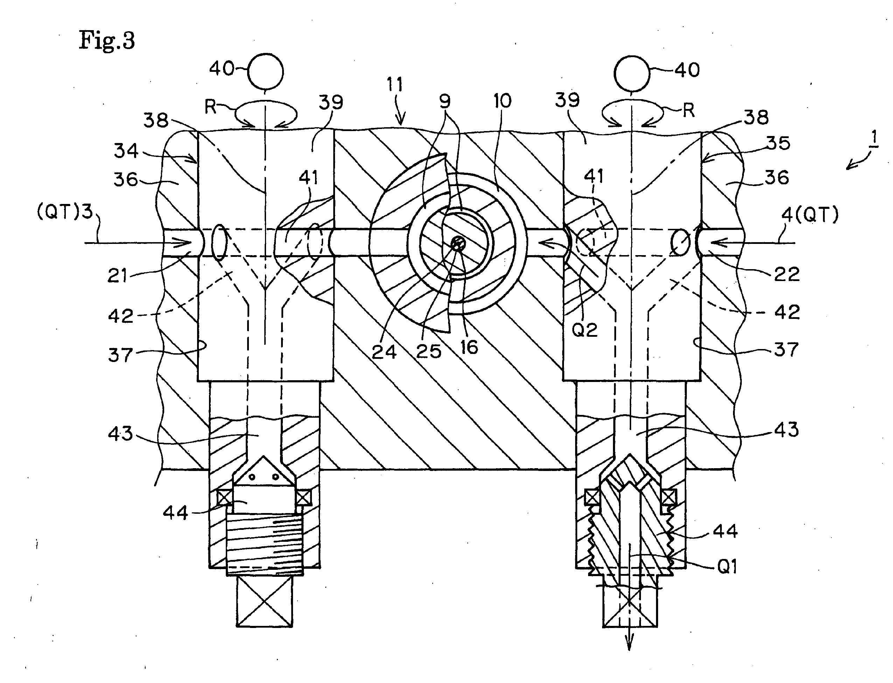

When the flow adjusting valves 34 and 35 are actuated by the driving of said actuators 40, said valve body 39 is turned as at R. And, when this valve body 39 is positioned at "full open position" (in Figs. 1 and 3, the state of the valve body 39 of the first flow adjusting valve 34), said first valve holes 41 provide communication between the divided ends of the inflow passages 21 and 22. Thereupon, the respective full flows (QT) of the resins 3 and 4 extruded from said extruders 6 and 7 are passed through said inflow passages 21 and 22 and the first valve holes 41 to said tube molding passages 9 and 10.

On the other hand, when the driving of said actuator 40 positions said valve body 39 at "half open position" (in Figs. 1 and 3, the state of the valve body 39 of the second flow adjusting valve 35), said second valve holes 42 provide communication between the divided ends of the inflow passages 21 and 22. And, partial flows (Q1) of the full flows (QT) of the resins 3 and 4 extruded from said extruders 6 and 7 pass through said communication passages 43 and opening-degree adjusting valves 44 and are discharged to outside the die 11, while remainder flows (Q2 = QT - Q1) pass through said inflow passages 21 and 22 and second valve holes 42 to said tube molding passages 9 and 10. In this case, the degree of opening of said communication passage 43 can be adjusted in advance by the operation of said opening-degree adjusting valve 44. This adjustment enables the adjustment of the remainder flows (Q2) passed to the tube molding passages 9 and 10.

Further, though not illustrated, when said valve bodies 39 are positioned at "full close position" by the driving of said actuators 40, the first and second valve holes 41 and 42 are closed by the inner peripheral surfaces of said valve body fit holes 37. In other words, said inflow passages 21 and 22 are fully closed. Thereupon, the flows of the resins 3 and 4 extruded from the extruders 6 and 7 and passed to the tube molding passages 9 and 10 are reduced to zero. In other words, in this manner, the degrees of opening of the inflow-passages 21 and 22 are made adjustable.

Further, as described above, when the valve bodies 39 are positioned at said "half open position" by the driving of the actuators 40, the communication passages 43 which communicate the intermediate portions of the inflow passages 21 and 22 to outside the die 11 are opened. When the valve bodies 39 are shifted from this state to said "full open position" or "full close position," the communication passages 43 are closed.

In the case of molding the multi-layer tube 2 by operating the extrusion molding apparatus 1, first, said extruders 6 and 7 and the take-up device 14 are driven. Further, in this case, the actuators 40 for the flow adjusting valves 34 and 35 are made able to be driven. The resins 3 and 4 extruded from the extruders 6 and 7 attending said driving pass through the inflow passages 21 and 22 and flow adjusting valves 34 and 35 to the inner and outer layer tube molding passages 9 and 10. And, the resins 3 and 4 are passed through the inner and outer layer tube molding passages 9 and 10 and extruded forwardly of the die 11, whereby the inner and outer layer tubes 2a and 2b are molded. Further, when these inner and outer layer tubes 2a and 2b are extruded from the extrusion ports 17 and 18, the outer layer tube 2b is externally fitted on the inner layer tube 2a and they are integrally fixed to each other, so that the multi-layer tube 2 is molded.

Further, simultaneously with the molding of the multi-layer tube 2, the core material 25 is forwardly passed through the through-hole 24. In the front vicinity of the extrusion ports 17 and 18 and the front end opening 26, the core material 25 has externally fitted thereon the inner layer tube 2a of the multi-layer tube 2, with the inner peripheral surface of this inner layer tube 2a being closely adhered thereto. Thereby, an intermediate product 47, which is a combination of the multi-layer tube 2 and the core material 25, is molded. This intermediate product 47 is passed through another die 30 with said die hole 29, whereby molding is effected such that any section of the multi-layer tube 2 along the longitudinal direction is perfectly circular and the outer diameter is constant. Further, thereafter, the intermediate product 47 is cold-cured by the cold curing device 13.

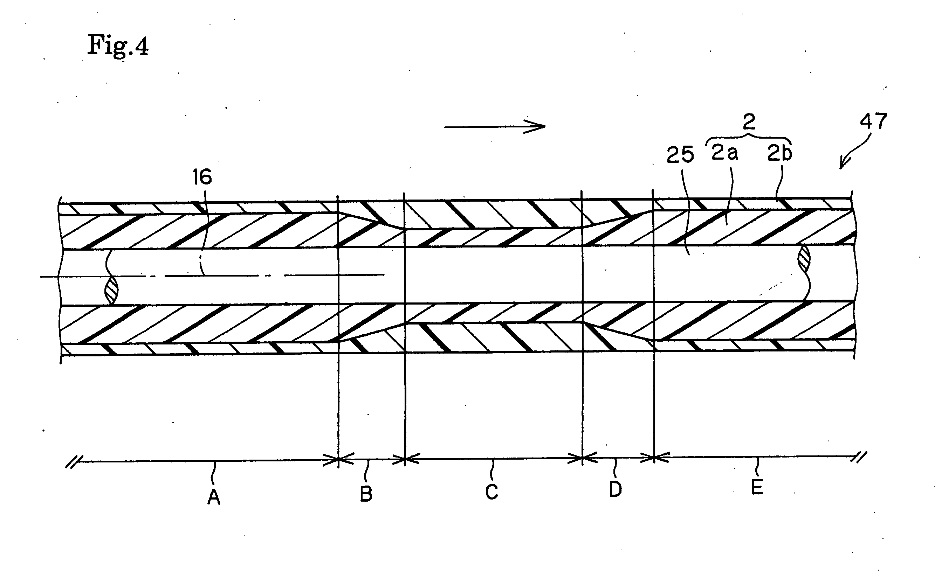

In Figs. 1 - 4, during molding of the intermediate product 47 by the extrusion molding apparatus 1, for example, as shown in Figs. 1 - 3, the valve body 39 of the first flow adjusting valve 34 is put in "full open position" and the valve body 39 of the second flow adjusting valve 35 is put in "half open position." Thereupon, the flow of the first resin 3 passed from the first extruder 6 through the first inflow passage 21 to the inner layer tube molding passage 9 is the full flow (QT) of the first resin 3 extruded from the first extruder 6, and increases more. On the other hand, the flow of the second resin 4 passed from the second extruder 7 through the second inflow passage 22 to the outer layer tube molding passage 10 is the remainder flow (Q2) of the second resin 4 extruded from the second extruder 7, and decreases more. Consequently, the multi-layer tube 2 molded in said state, as shown by A and E in Fig. 4, has its inner layer tube 2a thick-walled and its outer layer tube 2b thin-walled.

Reversely to the above, the valve body 39 of said first flow adjusting valve 34 is put in "half open position." Further, the valve body 39 of said second flow adjusting valve 35 is put in "full open position." Thereupon, by the action reverse to the above, the multi-layer tube 2, as shown by C in Fig. 4, has its inner layer tube 2a thin-walled and its outer layer tube 2b thick-walled.

As described above, in the case of putting the valve body 39 in "half open position," each of the flows of the resins 3 and 4 passed from the extruders 6 and 7 to the tube molding passages 9 and 10, is the reminder flow (Q2 = QT - Q1). However, since the partial flow (Q1) is discharged through said communication passage 43, the variation of the full flow (QT) of each of the resins 3 and 4 extruded from the extruders 6 and 7 is suppressed, and it is substantially constant. Here, when said valve body 39 is to be switched to either "full open position" or "half open position," more or less time is required for the turn R of the valve body 39. For this reason, as shown by B and D in Fig. 4, there are produced transition sections where the respective wall thicknesses of the inner and outer layer tubes 2a and 2b of the multi-layer tube 2 longitudinally change.

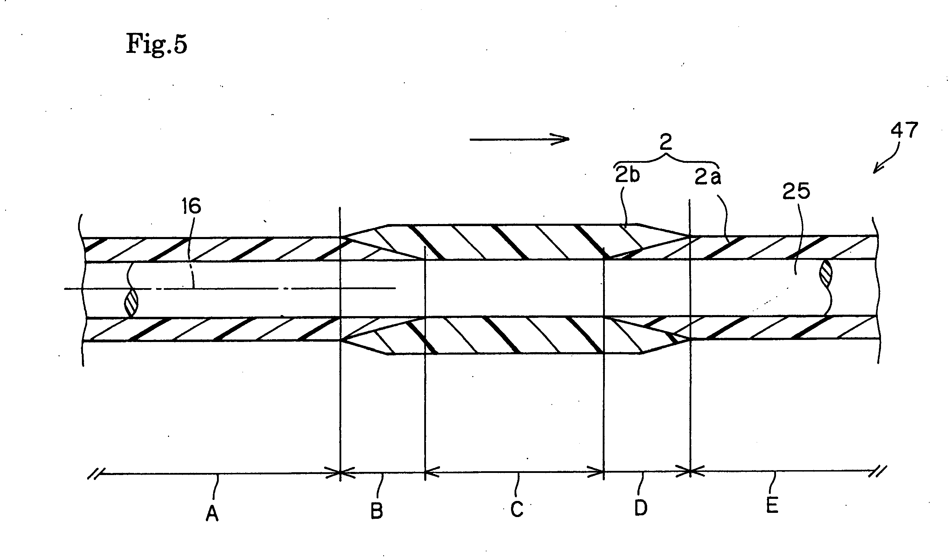

Referring to Figs. 1 - 3 and 5, during the molding of another intermediate product 47 by said extrusion molding apparatus 1, the valve body 39 of said first flow adjusting valve 34 is put in "full close position," with said another die 30 removed from said die 11. Further, the degrees of opening of said inflow passages 21 and 22 are adjusted to 0. Thereupon, as shown by A and E in Fig. 5, the multi-layer tube 2 is composed of the inner layer tube 2a alone. On the other hand, the valve body 39 of said first flow adjusting valve 34 is put in "full close position," while the valve body 39 of the second flow adjusting valve 35 is put in "full open position, and the degrees of opening of said inflow passages 21 and 22 are adjusted. Thereupon, as shown by C in Fig. 5, the multi-layer tube 2 is composed of the outer layer tube 2b alone.

In the above case, another die 30 does not exist. For this reason, the front vicinity of the extrusion port 18 constituting the front end of said outer layer tube molding passage 10 is opened radially outward of said axis 16. Consequently, the outer diameter of the outer layer tube 2b can be made greater than that of the inner layer tube 2a. In other words, the outer diameter of the multi-layer tube 2 can be adjusted to desired dimension at any longitudinal section. Further, the portions B and D in Fig. 5 are the same as the portions B and D of said Fig. 4.

Said intermediate product 47 is used as a material, for example, of catheters. That is, said intermediate product 47 is cut at predetermined longitudinal positions and into predetermined lengths by an unillustrated cutter. Thereafter, said core material 25 is longitudinally stretched by a stretching means, thereby being reduced in radial dimension. Then, this core material 25 is extracted from said multi-layer tube 2 so as to separate said core material 25 from the inner peripheral surface of the inner layer tube 2a of said multi-layer tube 2, whereupon said catheter is molded.

Here, the first resin 3 of which the inner layer tube 2a of said multi-layer tube 2 is molded differs in hardness from the second resin 4 of which the outer layer tube 2b is molded. For this reason, as shown in Figs. 4 and 5, the inner and outer layer tubes 2a and 2b in the multi-layer tube 2 have their respective wall thicknesses and radial dimensions radially adjusted. Thereupon, the hardness and shape at any section of said multi-layer tube 2 along the longitudinal direction can be continuously gradually changed, a fact which is convenient for molding catheters.

According to the above arrangement, inner and outer extrusion ports 17 and 18 constituting the respective front ends of the inner and outer layer tube molding passages 9 and 10 are disposed radially of said axis 16 and close to each other and opened forwardly of the front end surface 19 of the die 11 separately from each other.

For this reason, when the resins 3 and 4 are extruded from these extruders 6 and 7 by the driving of said extruders 6 and 7, these resins are passed through the tube molding passages 9 and 10 of said die 11, whereby inner and outer layer tubes are molded. Further, when said resins 3 and 4 are extruded from the inner and outer extrusion ports 17 and 18 forwardly of the die 11, the outer layer tube 2b is externally fitted integrally on said inner layer tube 2a, whereby a multi-layer tube is molded.

Here, as described above, the inner and outer extrusion ports 17 and 18 are disposed radially close to each other. For this reason, when said resins 3 and 4 are passed through the tube molding passage 9 and 10 of said die 11 and extruded forwardly from the inner and outer extrusion ports 17 and 18, said inner and outer layer tubes 2a and 2b immediately after they are extruded forwardly from said inner and outer extrusion ports 17 and 18 fit together and smoothly integrated without requiring relatively large radial deformation.

Furthermore, the inner and outer extrusion ports 17 and 18 are opened forwardly from the front end surface 19 of said die 11 separately from each other, as described above. For this reason, when said inner and outer layer tubes 2a and 2b fit together, these inner and outer layer tubes 2a and 2b are suppressed from pressing each other. Consequently, these inner and outer layer tubes 2a and 2b are prevented from being unintentionally deformed by their pressing each other.

As a result, the respective wall thicknesses of said inner and outer layer tubes 2a and 2b in the multi-layer tube 2 molded by said extrusion molding apparatus 1 can be made more accurate.

Further, said inner and outer extrusion ports 17 and 18 extend substantially in parallel with each other along the direction of said axis 16.

For this reason, when said inner and outer layer tubes 2a and 2b immediately after they are forwardly extruded from said inner and outer extrusion ports 17 and 18 fit together, these inner and outer layer tubes 2a and 2b are reliably suppressed from pressing each other. Consequently, these inner and outer layer tubes 2a and 2b are prevented from being unintentionally deformed by their pressing each other. As a result, the respective wall thicknesses of said inner and outer layers 2a and 2b can be made further accurate.

Further, said die 11 formed with a through-hole 24 longitudinally extending through said die 11 and inwardly of said inner layer tube molding passage 9, said multi-layer tube 2 being externally fitted on a core material 25 forwardly passing through said through-hole 24, and said inner extrusion port 17 of said inner layer tube molding passage9 is disposed close to the front end opening 26 constituting the front end of said through-hole 24 radially of said axis 16.

For this reason, when a multi-layer tube 2 is molded as it is extruded from said die 11 by the driving of said extruders 6 and 7, this multi-layer tube 2 is externally fitted on said front end opening 26, so that an intermediate product 47 is molded using the multi-layer tube 2 and the core material 25.

Here, the inner extrusion port 17 is disposed radially close to said front end opening 26, as described above. Furthermore, the inner and outer extrusion ports 17 and 18 are disposed radially close to each other. For this reason, when said multi-layer tube 2 is extruded forwardly of said die 11, the inner and outer layer tubes 2a and 2b immediately after their extrusion from said extrusion port 17 are, without requiring large radial deformation, externally fitted on the core material 25 immediately after slipping out of the front end opening 26 as it forwardly passes through said through-hole 24.

Consequently, the multi-layer tube 2 in said intermediate product 47 molded by said extrusion molding apparatus 1 can have the respective wall thicknesses of the inner and outer layer tubes 2a and 2b made more accurate.

Further, as described above, another die 30 is installed which is disposed forwardly of the die 11 and has a die hole 29 for communicating with said inner and outer extrusion ports 17 and 18.

For this reason, as shown in Fig. 4, the multi-layer tube 2 extruded from said die11 is passed through said die hole 29, whereby the perfect circularity and radial dimensions of said multi-layer tube 2 can be finally determined. Consequently, the respective wall thicknesses of the inner and outer layer tubes 2a and 2b in said multi-layer tube 2 can be made more uniform with respect to each other in any peripheral portion, so that the multi-layer tube 2 can be made more accurate.

Further, as described above, the front vicinity of said outer extrusion port 18 of the outer layer tube molding passage 10 is opened radially outward of said axis 16.

For this reason, as shown in Fig. 5, the outer diameter of the multi-layer tube 2 extruded from said die 11 can be optionally set in any section along the longitudinal direction, thus enabling the molding of multi-layer tubes 2 of various shapes.

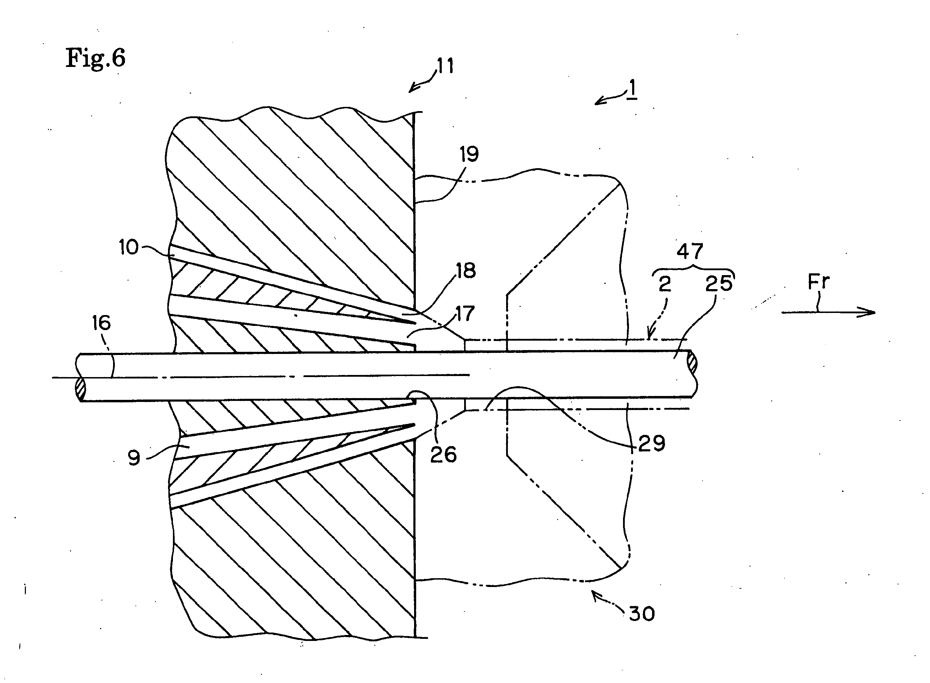

Fig. 6 shows another embodiment of said inner and outer extrusion ports 17 and 18.

According to this, said inner and outer extrusion ports 17 and 18 gradually approach each other radially of said axis 16 as they extend forward.

For this reason, when said inner and outer layer tubes 2a and 2b immediately after they are extruded forwardly from said inner and outer extrusion ports 17 and 18 fit together, these inner and outer layer tubes 2a and 2b are quickly joined, integration due to mutual fixing is achieved more reliably.

In addition, what has so far been described is by way of illustrated examples. Said multi-layer tube 2 and tube molding passages 9 and 10 may be three- or more-layered. Further, any one of the inner and outer layer tubes 2a and 2b of the multi-layer tube 2 may have its hardness increased. Further, a gear pump may be interposed between said extruders 6 and 7 and die 11. Further, said flow adjusting valves 34 and 35 may be interposed between said extruders 6 and 7 and die 11.

Further, the invention may be achieved by suitably combining individual component members described above.

## Claims

1. An extrusion molding apparatus for a resin multi-layer tube, comprising a plurality of extruders (6, 7) for thermally melting and extruding resins (3, 4) of different kinds, and a die (11) formed with an inner layer tube molding passage (9) for passing therethrough the resin (3) extruded from one extruder (6) of these extruders (6, 7) to enable the molding of an inner layer tube (2a), and an outer layer tube molding passage (10) for passing therethrough the resin (4) extruded from the other extruder (7) to enable the molding of an outer layer tube (2b) which is to be externally fitted integrally on said inner layer tube (2a), said die (11) enabling the molding of a multi-layer tube (2) by these inner and outer layer tubes (2a, 2b), said extrusion molding apparatus for resin multi-layer tube being **characterized in that** inner and outer extrusion ports (17, 18) constituting the respective front ends of said inner and outer layer tube molding passages (9, 10) are disposed radially close to each other and are opened forwardly of the front end surface (19) of the die (11) separately from each other.

2. In an extrusion molding apparatus for a resin multi-layer tube, with said die (11) formed with a through-hole (24) longitudinally extending through said die (11) and inwardly of said inner layer tube molding passage (9), said multi-layer tube being externally fitted on a core material (25) forwardly passing through said through-hole (24),
an extrusion molding apparatus as set forth in Claim 1, **characterized in that** said inner extrusion port (17) of said inner layer tube molding passage (9) is disposed radically close to the front end opening (26) radially constituting the front end of said through-hole (24).

3. An extrusion molding apparatus for a resin multi-layer tube, as set forth in Claim 1 or 2, **characterized in that** another die (30) is installed which is disposed forwardly of said die (11) and having a die hole (29) communicating with said inner and outer extrusion ports (17, 18).

4. An extrusion molding apparatus for a resin multi-layer tube, as set forth in Claim 1 or 2, **characterized in that** the front vicinity of said outer extrusion port (18) of said outer layer tube molding passage (10) is radially outwardly opened.
